# EUROPEAN PATENT APPLICATION

(11) **EP 4 672 043 A1**
(43) Date of publication of application: **31.12.2025**
(21) Application number: 24184810.0
(22) Date of filing: 26.06.2024
(51) Int. Cl.: G06F 18/2431, G06T 7/11, G06V 10/26, G06V 10/764

(54) **GENERATING SEGMENTATION MASK DATA FOR MEDICAL IMAGING DATA**

(71) Applicant: Siemens Healthineers AG, 91301 Forchheim (DE)
(72) Inventor: YEREBAKAN, Halid, Carmel, IN, 46033 (US); HERMOSILLO VALADEZ, Gerardo, West Chester, PA, 19382 (US)
(74) Representative: EIP

(57) **Abstract**

Disclosed is a method of generating segmentation mask data for first medical imaging data. The method comprises obtaining a first descriptor for a first location in the first medical imaging data, the first descriptor being representative of values of elements of the first medical imaging data located relative to the first location according to a first predefined pattern; determining, based on an input of the first descriptor to a trained machine learning model, a class label for each of a plurality of regions having a respective different predetermined location relative to the first location, the class label for each one of the plurality of regions being determined using a respective different one of a plurality of classifiers of the trained machine learning model; and generating the segmentation mask data for the first medical imaging data based on the class labels determined for the plurality of regions. Apparatus is also disclosed.

## Description

### Technical field

The present invention relates to a method, apparatus, and computer program for generating segmentation mask data for medical imaging data.

### Background

Medical images can be segmented to identify regions of interest, such as organs or medical abnormalities. For example, for a given medical image, a segmentation mask can be generated that shows the regions of the medical image where certain medical features, such as organs, are shown. Segmentation can, among other things, allow quantitative data to be obtained from a medical image (e.g. the size of a certain medical feature), enable radiotherapy to be precisely planned, and enable the identification of features, such as medical abnormalities, that might not be otherwise noticed by a medical professional.

Existing segmentation algorithms can be relatively slow and/or provide segmentation with limited resolution, which can limit their utility. It is therefore desirable to improve the speed at which and/or the resolution with which a segmentation mask can be generated for a medical image. For example, increasing the speed with which a segmentation mask of a given resolution can be generated may, for example, in turn improve the speed and flexibility with which a medical professional can utilize the segmentations mask.

### Summary

According to a first aspect of the present invention, there is provided a computer implemented method of generating segmentation mask data for first medical imaging data, the first medical imaging data comprising an array of elements having respective values and representing respective locations, the method comprising: obtaining a first descriptor for a first location in the first medical imaging data, the first descriptor being representative of values of elements of the first medical imaging data located relative to the first location according to a first predefined pattern; determining, based on an input of the first descriptor to a trained machine learning model, a class label for each of a plurality of regions of the first medical imaging data, each region having a respective different predetermined location relative to the first location, the class label for each one of the plurality of regions being determined using a respective different one of a plurality of classifiers of the trained machine learning model; and generating the segmentation mask data for the first medical imaging data based on the class labels determined for the plurality of regions.

Optionally, the method comprises: by a projection component of the trained machine learning model, projecting the first descriptor or a descriptor derived from the first descriptor to each of a plurality of second descriptor spaces, thereby to determine a respective plurality of second descriptors; and wherein the class label for each one of the plurality of regions is determined based on an input of a respective one of the plurality of second descriptors into a respective one of the plurality of classifiers of the trained machine learning model.

Optionally, for each of the of the plurality of second descriptor spaces, the projection of the first descriptor or the descriptor derived from the first descriptor to the second descriptor space reduces the dimensionality of the first descriptor or the descriptor derived from the first descriptor.

Optionally, the method comprises: determining, based on an input of the first descriptor or the descriptor derived from the first descriptor to a first classifier of the trained machine learning model, a probability value for each one of a plurality of class labels; wherein determining the class label for each one of the plurality of regions using a respective different one of the plurality of classifiers of the trained machine learning model is responsive to each one of the determined probability values being less than a threshold; and wherein the method comprises: in response to a particular one of the determined probability values being greater than the threshold, assigning the class label for which the particular probability value was determined to each of the plurality of regions, thereby to determine the class label for each of the plurality of regions.

Optionally, the method comprises: determining, based on an input of the first descriptor into a residual neural network of the trained machine learning model, the descriptor derived from the first descriptor.

Optionally, the first predefined pattern is such that the density of elements represented by the first descriptor decreases with increasing distance from the first location.

Optionally, obtaining the first descriptor comprises: obtaining first pattern data indicating distances from the first location of elements to be represented in first descriptor; converting, based on scaling data indicative of the size of space that each element represents, the distances to element offsets; and for each of the element offsets, determining the value of the element of the first medical imaging data at the element offset, thereby to obtain the first descriptor.

Optionally, the method comprises: performing the steps of obtaining the first descriptor and determining the class label for each of the plurality of regions, for each of a plurality of different first locations in the first medical imaging data in parallel, thereby to determine the class label for each region of a respective plurality of sets of regions of the first medical image data; and generating the segmentation mask data based on the class labels determined for each region of the plurality of sets of regions.

Optionally, the segmentation mask data comprises an array of elements each having a respective segmentation mask value and representing respective locations, wherein, for each element, the segmentation mask value represents the class label determined for the region in which the element is located.

Optionally, the method comprises: storing the segmentation mask data in a storage device and/or displaying a segmentation mask rendered from the segmenting mask data on a display device.

Optionally, the trained machine learning model has been trained by a training method comprising: providing a machine learning model configured to performs steps comprising: based on an input of a given descriptor for a given location in given medical imaging data, determining a class label for each of a plurality of regions of the given medical imaging data, each region having a respective different predetermined location relative to the given location, the class label for each one of the plurality of regions being determined using a respective different one of a plurality of classifiers of the machine learning model; providing training data comprising: a plurality of training descriptors, each training descriptor being for a respective given location in given training medical imaging data, each training descriptor being representative of values of elements of the given training medical imaging data located relative to the given location according to the first predefined pattern; and for each training descriptor, a corresponding ground truth class label for each of the plurality of regions of the given training medical imaging data; and training the machine learning model based on the training data, wherein the training comprises modifying parameters of the classifiers to minimize a loss function between the class labels determined by the classifiers based on the training descriptors and the corresponding ground truth class labels.

Optionally, the provided machine learning model is configured to perform steps comprising: by a projection component of the machine learning model, projecting the given descriptor or a descriptor derived from the given descriptor to each of a plurality of second descriptor spaces, thereby to determine a respective plurality of second descriptors; wherein the class label for each one of the plurality of regions is determined based on an input of a respective one of the plurality of second descriptors into a respective one of the plurality of classifiers of the machine learning model; and wherein the training comprises modifying parameters of the projection component to minimise the loss function between the class labels determined by the classifiers based on the training descriptors and the corresponding ground truth class labels.

Optionally, the provided machine learning model is configured to perform steps comprising: determining, based on an input of the given descriptor, or the descriptor derived from the given descriptor, to a first classifier, a probability value for each one of a plurality of class labels; wherein the training data comprises: for each training descriptor, a corresponding ground truth probability value for each of the plurality of class labels; and wherein training the machine learning model comprises: modifying parameters of the first classifier to minimize a loss function between the probability values determined by the first classifier based on the training descriptors and the corresponding ground truth probability values.

Optionally, the provided machine learning model is configured to perform steps comprising: determining, based on an input of the given descriptor into a residual neural network of the machine learning model, the descriptor derived from the given descriptor; wherein the training comprises modifying parameters of the residual neural network so as to minimize the loss function between the class labels determined by the classifiers based on the training descriptors and the corresponding ground truth class labels; and/or wherein the training comprises modifying parameters of the residual neural network so as to minimise the loss function between the probability values determined by the first classifier based on the training descriptors and the corresponding ground truth probability values.

According to a second aspect of the invention, there is provided a training method comprising: providing a machine learning model configured to performs steps comprising: based on an input of a given descriptor for a given location in given medical imaging data, determining a class label for each of a plurality of regions of the given medical imaging data, each region having a respective different predetermined location relative to the given location, the class label for each one of the plurality of regions being determined using a respective different one of a plurality of classifiers of the machine learning model; providing training data comprising: a plurality of training descriptors, each training descriptor being for a respective given location in given training medical imaging data, each training descriptor being representative of values of elements of the given training medical imaging data located relative to the given location according to a first predefined pattern; and for each training descriptor, a corresponding ground truth class label for each of the plurality of regions of the given training medical imaging data; and training the machine learning model based on the training data, wherein the training comprises modifying parameters of the classifiers to minimize a loss function between the class labels determined by the classifiers based on the training descriptors and the corresponding ground truth class labels.

According to a third aspect of the present invention, there is provided apparatus configured to perform the method according to the first aspect or the second aspect.

According to a fourth aspect of the present invention, there is provided a computer program which when executed by a computer causes the computer to perform the method according to the first aspect or the second aspect.

### Brief description of the drawings

Figure 1 is a flow diagram illustrating a method of generating segmentation mask data for first medical imaging data, according to an example;
Figure 2 is a schematic diagram illustrating first medical imaging data and a first predefined pattern, according to an example;
Figure 3a is a schematic diagram illustrating first medical imaging data and a first predefined pattern, according to another example;
Figure 3b is a schematic diagram illustrating a visualisation of a first descriptor, according to an example;
Figure 4 is a flow diagram illustrating a method of generating segmentation mask data for first medical imaging data, according to an example;
Figure 5 is a flow diagram illustrating a method of generating segmentation mask data for first medical imaging data, according to an example;
Figure 6 is a flow diagram illustrating a method of generating segmentation mask data for first medical imaging data, according to an example;
Figure 7 is a schematic diagram illustrating segmentation mask data according to an example;
Figure 8 is a flow diagram illustrating a method of training a machine learning model, according to an example; and
Figure 9 is a schematic diagram illustrating a system and apparatus according to an example.

### Detailed description

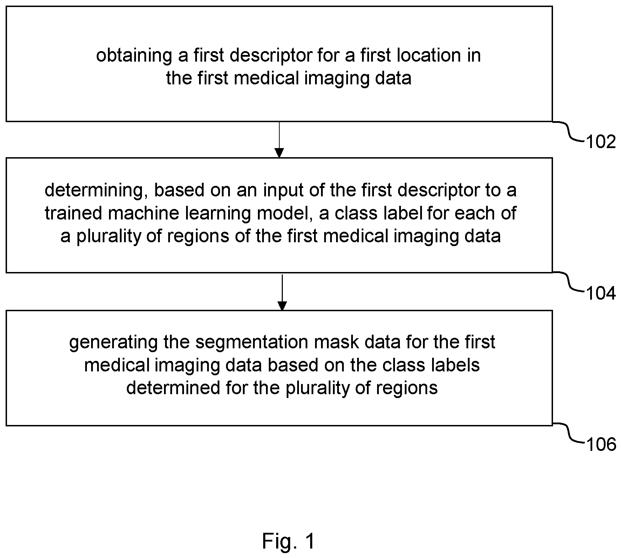

Figure 1 shows a flow diagram of a computer implemented method of generating segmentation mask data 570 for first medical imaging data 202, 302, 502. The first medical imaging data comprises an array of elements having respective values and representing respective locations. In broad overview, the method comprises:
- in step 102, obtaining a first descriptor 332, 406, 506 for a first location 204, 304, 404, 538 in the first medical imaging data, the first descriptor being representative of values of elements 206, 306 of the first medical imaging data located relative to the first location according to a first predefined pattern 212, 312, 412;
- in step 104, determining, based on an input of the first descriptor to a trained machine learning model 460, 560, a class label A, B, C for each of a plurality of regions 430-436, 530-536 of the first medical imaging data, each region having a respective different predetermined location relative to the first location 404, 538, the class label for each one of the plurality of regions being determined using a respective different one of a plurality of classifiers 442-448, 552-558 of the trained machine learning model; and
- in step 106, generating the segmentation mask data 570 for the first medical imaging data based on the class labels determined for the plurality of regions.

Accordingly, the class labels for a plurality of regions having respective predetermined locations relative to the first location are determined based on the descriptor for the first location. As such, a different first descriptor need not be obtained for each of the predetermined locations. This can allow for the speed with which the segmentation mask data can be generated to be increased, especially in cases where processing of the first descriptor is resource intensive. Further, the class label for each one of the plurality of regions is determined using a respective one of a plurality of classifiers of a trained machine learning model. As such, the class labels need not necessarily be determined one at a time and/or the class label for one region need not necessarily be dependent on the class label for another region. Instead, the class labels for each of the plurality of regions can be determined simultaneously. This can increase the speed with which the segmentation mask can be generated. Alternatively, or additionally, as such, the same class label need not necessarily be assigned to each of the regions, and instead different class labels can be assigned to different regions as appropriate. This can allow for a segmentation mask with relatively high resolution. Accordingly, the method may provide for an improvement in the speed at which and/or the resolution with which segmentation mask data can be generated for a medical image. In other words, the method may provide for fast generation of segmentation mask data having a relatively high resolution. As such, the method may provide for improved segmentation mask data generation.

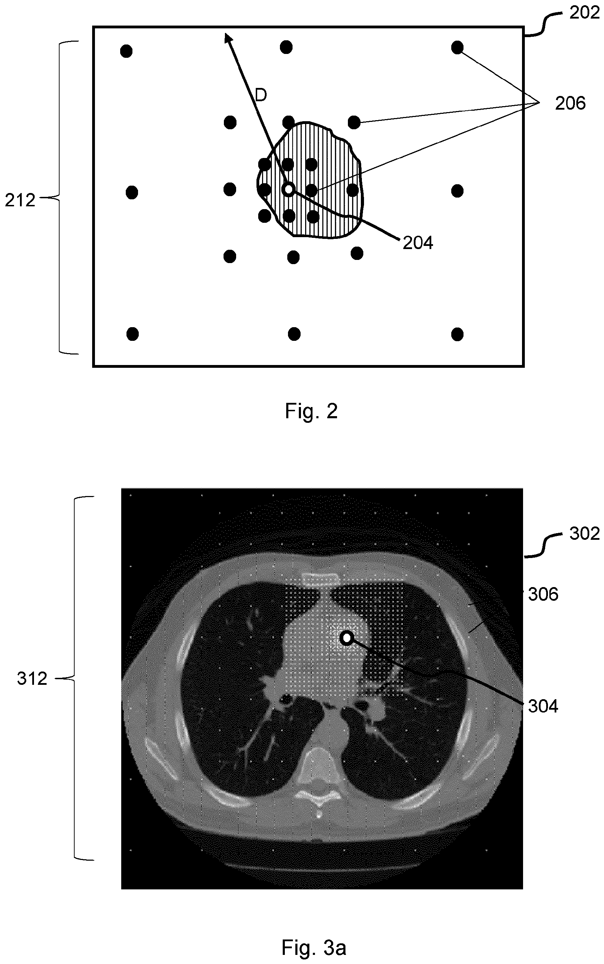

As mentioned, the method is a method of generating segmentation mask data for first medical imaging data. Examples of medical imaging data are illustrated in Figures 2 and 3a. The medical imaging data may be captured by performing medical imaging on a patient, for example Computed Tomography (CT), Magnetic Resonance Imaging (MRI), X-ray, or other imaging techniques. Figures 2 and 3a each illustrate a representation of medical imaging data 202, 302. In each case, the medical imaging data comprises an array of elements each having respective values and representing respective locations. For example, the medical imaging data may comprise a 2-Dimensional array of pixels, each pixel having at least one value, and each pixel representing a location in a 2-Dimensional imaging plane. As another example, the medical imaging data may comprise a 3-Dimensional array of voxels, each voxel having at least one value, and each voxel representing a location in 3-Dimensional space. The at least one value may correspond to or otherwise be representative of an output signal of the medical imaging technique used to generate the medical imaging data. For example, for X-ray imaging, the value of an element (e.g. pixel) may correspond to or represent a degree to which X-rays have been detected at the particular part of the imaging plane corresponding to the element. As another example, for Magnetic Resonance Imaging, the value of an element (e.g. voxel) may correspond to or represent a rate at which excited nuclei, in a region corresponding to the element, return to an equilibrium state. In some examples, each element may only have one value. However, in other examples, each element may have or otherwise be associated with multiple values. For example, the multiple values of a given element may represent the values of respective multiple signal channels. For example, each signal channel may represent a different medical imaging signal or property of the imaging subject. In some examples, the at least one value may comprise an element (e.g. pixel or voxel) intensity value. For example, an output signal from the medical imaging may be mapped onto a pixel or voxel intensity value, for example a value within a defined range of intensity values. For example, for a greyscale image, the intensity value may correspond to a value in the range 0 to 255, where 0 represents a 'black' pixel and 255 represents a 'white' pixel, for example. As another example, for example as in the case of USHORT medical image data, the intensity value may correspond to a value in the range 0 to 65536. As another example, in a color image (e.g. where different colors represent different properties of the imaging subject) each pixel/voxel may have three intensity values, e.g. one each for Red, Green, and Blue channels. It will be appreciated that other values may be used. In any case, the medical imaging data may be rendered into an image, for example as schematically illustrated in Figures 2 and 3a.

As mentioned, the method of Figure 1 comprises in step 102, obtaining a first descriptor for a first location 204, 304 in the first medical imaging data 202, 302. The first descriptor is representative of values of elements 206, 306 of the first medical imaging data 204, 304 located relative to the first location according to a first predefined pattern 212, 312.

In some examples, the first descriptor may be output from a descriptor model applied to the first medical imaging data 202, 302 for the first location 204, 304. The descriptor model may be configured to determine a descriptor for a given location based on the values of elements located relative to the given location according to the first predefined pattern. 212, 312

In some examples, the first descriptor may be obtained from a database (not shown). For example, the descriptor for the first location 204, 304 may have already been calculated (for example by applying the descriptor model), and stored in the database, for example in association with the first location 204, 304. For example, the database may store a plurality of first descriptors each in association with the corresponding first location in the medical imaging data on the basis of which the first descriptor was determined. Accordingly, in some examples, the method may comprise selecting the first location 204, 304 from among the plurality and extracting the first descriptor associated with the selected first location 204, 304.

In either case, a descriptor for a given location may be a vector comprising a plurality of entries, each entry being representative of the value (e.g. an intensity value) of an element (e.g. a pixel or voxel), the elements being located relative to the given location according to the first predefined pattern 212, 312. The first predefined pattern 212, 312 may be, for example, a grid-like pattern, such as the grid-like pattern 212 shown in Figure 2 or the grid-like pattern 312 shown in Figure 3a. In some examples, the descriptor may be determined using many such values of elements, for example hundreds or thousands of elements, and accordingly the descriptor may be a vector having many entries (e.g. hundreds of thousands of entries). For example, referring to Figure 2, there is presented, for illustrative purposes, a medical imaging data set 202 to which a complex grid containing a large number of element locations (shown as black dots) 206 has been applied in order to determine a descriptor for a given location 204 at the center of this grid. As can be seen, the density of element locations 206 in the predefined pattern 212 decreases as the distance D from the given location 204 increases. As another example, referring to Figure 3a, there is presented, for illustrative purposes, a medical imaging data set 302 to which a complex grid containing a large number of element locations (shown as white dots) 306 has been applied in order to determine a descriptor for a given location 304 at the center of this grid 312. As can be seen, the density of locations 306 in the predefined pattern 312 decreases as the distance from the given location 204 increases.

The first descriptor may encode the spatial context of the first location 204, 304, and hence in turn may provide a compact representation of the first location 204, 304 and its surroundings. The first descriptor may provide a sparse encoding of the spatial context of the first location 204, 304. For example, as above, the first predefined pattern may be such that the density of elements 206, 306 represented by the first descriptor decreases with increasing distance from the first location 204, 304. This may allow for both a 'wide field of view' to allow the first descriptor to sparsely represent the wider context of the first location 204, 304, as well as a 'narrow field of view' to allow the first descriptor to represent more densely the detail close to the first location 204, 304. This may allow for accurate segmentation, but using a sparse descriptor, which may improve processing speed. For example, in the examples of Figure 2 and 3a, the density of element locations 206, 306 relatively near to the first location 204, 304 is relatively high. This provides a relatively fine-grained and detailed encoding of the spatial context relatively near the first location 204, 304. On the other hand, the density of element locations 206, 306 relatively distant from the first location 204, 304 is relatively low. This provides a relatively sparse encoding of the spatial context relatively distant from the first location 204, 304, and hence provides a wide field of view of the spatial context. The spatial context relatively distant from the first location 204, 304 is likely to be less important to the segmentation of regions near the first location 204, 304, and hence is encoded more sparsely, but nonetheless may still be encoded as including this distant spatial context can nonetheless improve the accuracy of segmentation near the first location 204, 304.

In examples, the first pattern 212, 312 may comprise element locations 206, 306 each having respective different predefined offsets relative to the first location 204, 304. For example, each element location 206, 306 of the first predefined pattern 212, 312 may be defined according to a respective different element offset from the first location (e.g. 3 pixels/voxels to the right of the first location). In some examples, each element location 206, 306 of the first predefined pattern 212, 312 may be defined according to a respective different spatial offset from the first location (e.g. 3 mm to the right of the first location). This may help ensure that the first descriptor can encode the same spatial context across different medical images having different scaling. In this case, applying the descriptor model may comprise converting the defined spatial offsets of the first predefined pattern to corresponding element offsets from the first location 204, 304. For example, this may be achieved using scaling data included with the first medical imaging data, for example in a header of a the first medical imaging data. For example, the scaling data may indicate that the length of each element (e.g. pixel) of the first medical imaging data 202, 302 corresponds to 1mm. Accordingly, in examples, obtaining the first descriptor may comprise obtaining first pattern data indicating distances (e.g. in mm) from the first location 204, 304 of elements 206, 306 to be represented in first descriptor; converting, based on scaling data indicative of the size of space that each element represents, the distances to element offsets (e.g. in pixels/voxels); and for each of the element offsets, determining the value of the element of the first medical imaging data located at the element offset, thereby to obtain the first descriptor. This may allow for the same first descriptor to be applied independent of the scaling of the medical imaging data. This may help allow for accurate segmentation independent of the different scaling of different the medical image data being processed. This may, in turn, allow for accurate and flexible segmentation. Further, converting the distances to element offsets may provide for efficient determination of the first descriptor. For example, adding element offsets to the element at the first location, and then determining (e.g. looking up) the value of the corresponding element in the medical imaging data, may provide a computationally inexpensive, and hence efficient, way of determining the first descriptor.

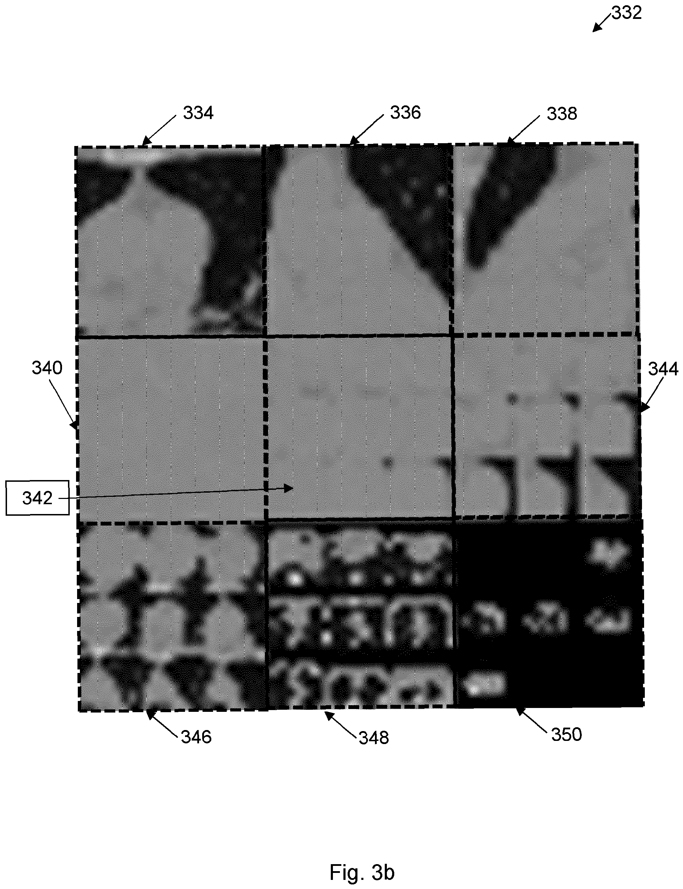

Referring to Figure 3b, there is illustrated a visualisation of a first descriptor 332 for the first location 304 of the medical imaging data 302 of Figure 3a, obtained by applying the first predefined pattern 312 of Figure 3a. In this example, the medical imaging data 302 is 3-Dimensional (only one slice is sown in Figure 3a). In this example, the first predefined pattern 312 comprises 2-Dimensial grids and 3-Dimensional grids. Specifically, in this example, the first predefined pattern 312 comprises three orthogonal 2-Dimensional grids, each having a 4mm grid resolution, and each comprising 27x27 elements. The first predefined pattern 312 also comprises six 3-Dimensional grids, having grid resolutions of 2, 3, 5, 12, 28 and 64 mm, respectively, and each comprising 9x9x9 elements. Referring to the visualisation of this descriptor in Figure 3b, the three boxes 334, 336, 338 represent values of the elements obtained by applying the three orthogonal 27x27 2-Dimensional grids, respectively. The six boxes 340, 342, 344, 346, 348, 350 represent the values of elements obtained by applying the six 9x9x9 3-Dimensional grids, respectively. Each of these six boxes 340, 342, 344, 346, 348, 350 respectively show 9 lots of 9x9 slices through the respective 3-D grid (in order to allow 2-Dimensional visualisation of the 3-Dimensional space represented by the 9x9x9 grids). In this example, the total dimension of the first descriptor is 6561.

It will be appreciated that, in some examples, descriptors other than the specific examples described above may be used. For example, different grid resolutions, sizes and shapes may be used. As another example, in some examples, each entry may be representative of the values of the elements located within a respective one or more of a plurality of predefined boxes (e.g. rectangular regions) located relative to the given location according to the first predefined pattern. It will be appreciated that, where the medical imaging data exists in three spatial dimensions, the term 'box' as used herein may refer to a cuboidal region or volume. In some examples, each entry of the descriptor may be representative of the values of the elements located within a respective one of a plurality of predefined boxes. For example, each entry of the descriptor may be an average of the values of the elements located within a respective one of a plurality of predefined boxes. That is, each entry may be the sum of the values of the elements located within a particular box, divided by the number of elements included in the box. Nonetheless, the descriptor for a given location is representative of values of elements of the medical imaging data located relative to the given location according to a first predefined pattern. Other descriptors may be used. It will be appreciated that, where the medical imaging data is 2-Dimensional, the first predefined pattern 212, 312 may be 2-Dimensional, and in cases where the medical imaging data is 3-Dimensional, the first predefined pattern 212, 312 may be 3-Dimensional and/or 2-Dimensional.

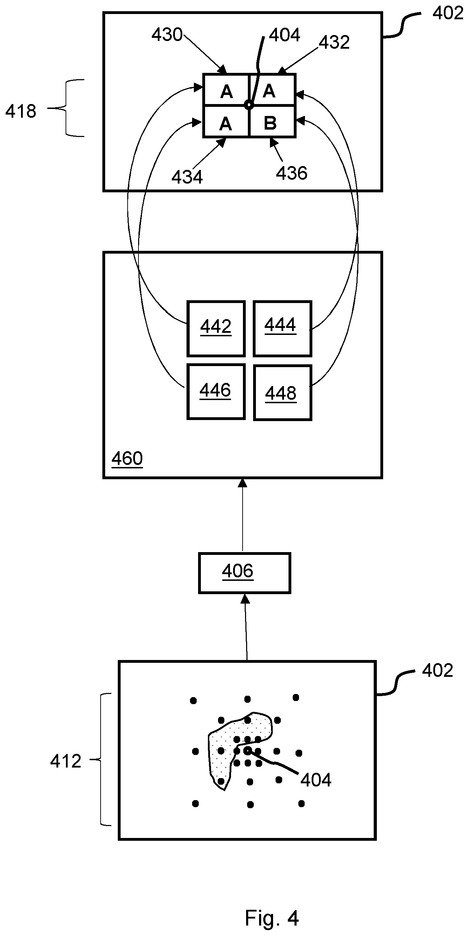

As mentioned, the method of Figure 1 comprises, in step 104, determining, based on an input of the first descriptor to a trained machine learning model, a class label for each of a plurality of regions of the first medical imaging data, each region having a respective predetermined location relative to the first location, the class label for each one of the plurality of regions being determined using a respective different one of a plurality of classifiers of the trained machine learning model. Referring to Figure 4, there is illustrated a trained machine learning model 460 comprising a plurality of classifiers 442, 444, 446, 448, according to an example. For example, the trained machine learning model 460 may be a neural network. In the example of Figure 4, as above, a first descriptor 406 is obtained for a first location 404 in the first medical imaging data 402. Specifically, as above, the first descriptor 406 is representative of values of elements of the first medical imaging data 402 located relative to the first location 404 according to a first predefined pattern 412.

Based on an input of the first descriptor 406 to the trained machine learning model 460, the trained machine learning model 460 determines a class label A, B for each of a plurality of regions 430, 432, 434, 436 of the first medical imaging data 402. For example, the class label may be an organ label, such as 'liver', 'kidney', 'heart', 'lung' or the like, of the organ represented at each one of the regions. In examples, each region may be a single element (e.g. pixel or voxel) of the first medical imaging data. In other examples, each region may cover a plurality of elements (e.g. pixels or voxels) such as a group of adjacent elements, of the first medical imaging data 402. Each region 430-432 has a respective predetermined location relative to the first location 404. For example, each region 430-432 may have a respective different predetermined offset relative to the first location 404. For example, the predetermined locations of the regions 430-436 relative to the first location 404 may be such that the regions together surround the first location 404. For example, the regions 430-436 may be the same size and shape (boxes) as one another. In the example of Figure 4, the regions 430-436 are adjacent to one another and together form a block 418, with the first location 404 being in the centre of the block 418. In the example of Figure 4, a first region 430 is offset above and to the left of the first location 404, a second region 432 is offset above and to the right of the first location 404, a third region 434 is offset below and to the left of the first location 404, and a fourth region 436 is offset below and to the right of the first location 404.

The class label A, B for each one of the plurality of regions 430, 432, 434, 436 is determined using a respective different one of a plurality of classifiers 443, 444, 446, 448 of the trained machine learning model 460. For example, for each region 430-436 for which a class label is to be determined, there may be a corresponding classifier 442-448 configured to determine, based on the first descriptor 406 or a descriptor derived from the first descriptor (described in more detail below), the class label A, B for that region 430-436. As such, there may be one classifier 442, 448 for each region 430-436. In other words, each different region 430-436 may correspond to a respective different classifier 442-448 that is configured to determine a class label for that region. Each classifier 442-448 may have learned, through the training of the machine learning model 460, to determine a class label for a respective region 430-436 having a particular predefined location (e.g. offset) relative to the first location 404. In other words, each classifier 442-448 may have learned to decode the first descriptor 406 (or a descriptor derived from the first descriptor 406) in a specific offset position or region relative to the first location 404. Accordingly, based on the first descriptor 406, the trained machine learning model 460 can independently determine a respective class label A, B for each respective one of the plurality of regions 430-436. Each classifier 442-448 may be configured to determine, for the corresponding region 430-436, the appropriate one of a plurality of possible class labels, such as one of a plurality of organ class labels, such as 'liver', 'kidney', 'heart', 'lung' and the like. In other words, each classifier 442-4488 may be a multi-class classifier (e.g. a multi-organ classifier). It will be appreciated that in examples there may be any number of classifiers 442-448 and a corresponding number of regions 430-436. As an example, for 3-Dimensioal medical imaging data, there may be 125 cuboidal regions arranged in a 3-Dimensional block of 5x5x5 regions having 2mm resolution. That is, each of the 125 regions may be 2mm³, together providing a block of 10mm³. Correspondingly the trained machine learning model may comprise 125 classifiers, one for each region. It has been found that this provides a useful balance between segmentation resolution and speed, but it will be appreciated that other numbers and arrangements of regions and classifiers may be used.

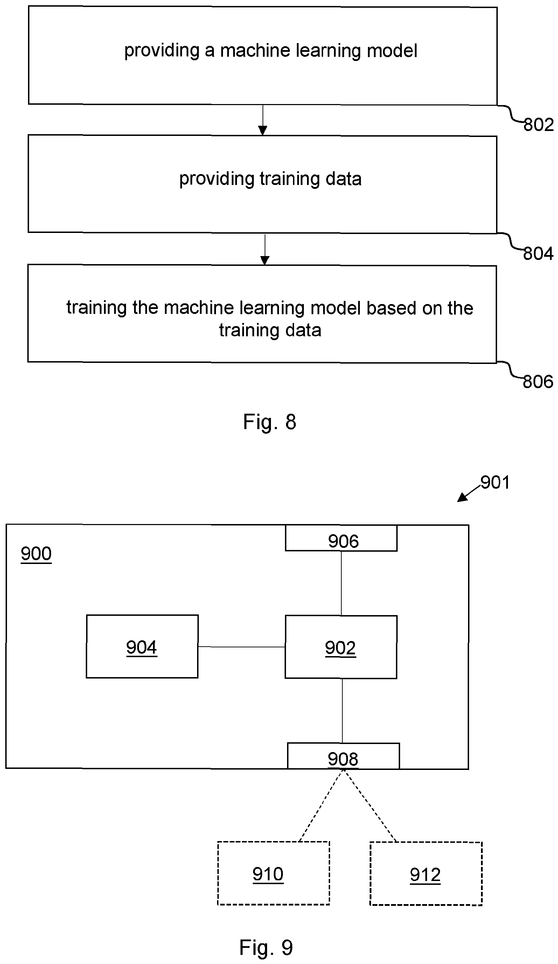

The trained machine learning model 460 may be trained using supervised learning. For example, the trained machine learning model 460 may be trained using a training method, such as the training method described below with reference to Figure 8. For example, the trained machine learning model 460 may have been trained by a training method comprising providing a machine learning model, providing training data, and training the machine learning model based on the training data. For example, the provided machine learning model (such as a neural network) may be configured to performs steps comprising (as per the trained machine learning model 460): based on an input of a given descriptor for a given location in given medical imaging data, determining a class label for each of a plurality of regions of the given medical imaging data, each region having a respective different predetermined location relative to the given location, the class label for each one of the plurality of regions being determined using a respective different one of a plurality of classifiers of the machine learning model. As above, each classifier may be configured to determine the appropriate class label for the corresponding region from a plurality of class labels, such as a plurality of organ labels, such as 'liver' 'kidney' `heart', `lung' and the like. The training data may comprise a plurality (for example hundreds or thousands) of training descriptors, each training descriptor being for a respective given location in given training medical imaging data, each training descriptor being representative of values of elements of the given training medical imaging data located relative to the given location according to the first predefined pattern. The training descriptors may be obtained from respective sets of training medical imaging data. The training descriptors may be obtained using the same descriptor model that is used to obtain the first descriptor, as described above. The training data may also comprise, for each training descriptor, a corresponding ground truth class label (e.g. organ label) for each of the plurality of regions of the given training medical imaging data. For example, the ground truth class labels may be provided by annotation of the given training medical imaging data by a medical professional, although other annotation mechanisms are possible. Training the provided machine learning model based on the training data may comprise modifying parameters of the classifiers (for example weights of neurons of one or more neural network layers of each classifier) to minimize a loss function between the class labels determined by the classifiers based on the training descriptors and the corresponding ground truth class labels. For example, the loss function may comprise the categorical cross-entropy loss or cross-entropy loss between the class labels predicted by the classifiers based on input of the training descriptors and the corresponding ground truth class labels of the training data. Other loss functions may be used, such as Focal loss, or Dice loss. Accordingly, the trained machine learning model 460 can be provided.

As mentioned, the method of Figure 1 comprises, in step 106, generating segmentation mask data for the first medical imaging data based on the class labels determined for the plurality of regions. In examples, the segmentation mask data may comprise an array of elements each having a respective segmentation mask value and representing respective locations. For each element, the segmentation mask value may represent the class label A, B determined for the region 430-432 in which the element is located. For example, the array of elements of the segmentation mask data may be the same as the array of elements of the first medical imaging data.

In examples, the segmentation mask data may be added to the first medical imaging data 402. For example, for each element of the first medical imaging data 402, the class label of the region 430-436 in which the element is located may be added as a segmentation mask value for that element. This may allow, for example, a rendering of the medical image and/or a segmentation mask for the medical image, from a single set of medical imaging data 402.

In examples, the segmentation mask data may be stored in a separate data structure to the medical imaging data 402. For example, the segmentation mask data may comprise an array of elements each representing a respective location (e.g. pixels or voxels). For example, the array of elements of the segmentation mask data may be the same (e.g. the same number and arrangement) as the array of elements of the first medical imaging data 402 for which the segmentation mask data is generated. In this case, each element of the segmentation mask data may correspond to a specific element of the first medical imaging data 402. In this case, generating the segmentation mask data may comprise, for each element of the segmentation mask data, setting the value of the element as the class label determined for the region in which the corresponding element of the first medical imaging data 402 is located. A segmentation mask may be generated from the segmentation mask data and/or a medical image may be generated from the first medical image data. The segmentation mask and the medical image may be overlayed or otherwise combined to allow, for a given location in the medical image, a determination of the class label (e.g. organ label) for that location.

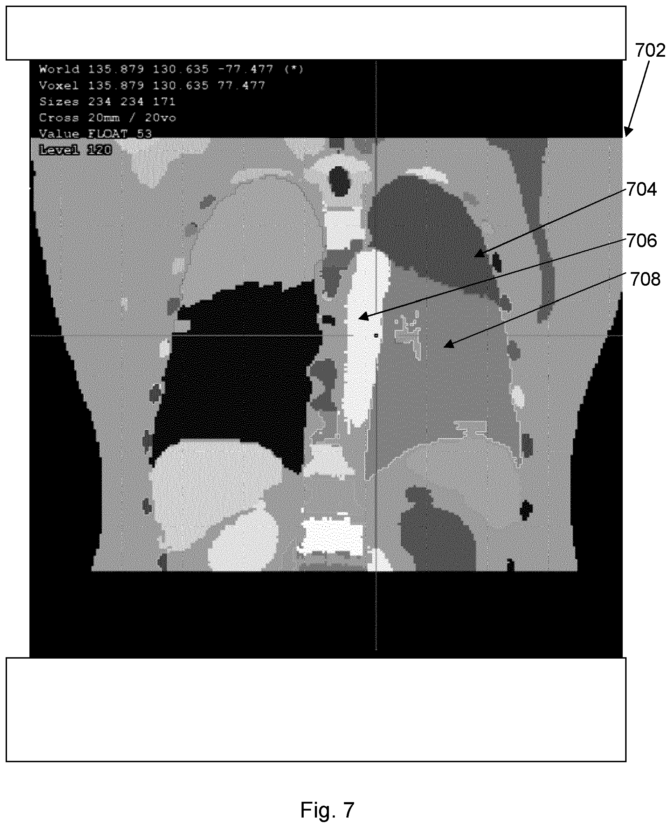

In examples, the method may comprise storing the segmentation mask data in a storage device (not shown) such as a memory or a database, and/or displaying a segmentation mask rendered from the segmenting mask data on a display device (not shown) such as a computer monitor. Referring briefly to Figure 7 (described in more detail below), there is illustrated a segmentation mask 702 rendered from segmentation mask data according to an example. The generated segmentation mask data can be rendered to display the segmentation mask (e.g. an organ, or other classification, segmentation mask) for the first medical image for which the segmentation mask data is generated.

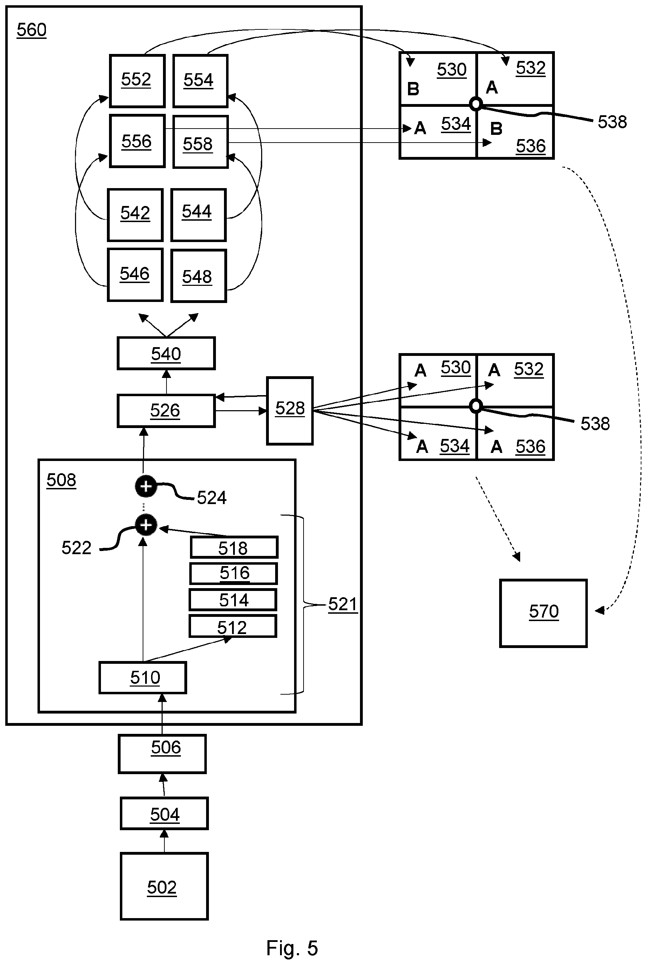

Figure 5 illustrates an example of the method described above with reference to Figure 1. The example method of Figure 5 includes certain features which may further improve the segmentation mask generation, for example further improve the speed at and/or accuracy with which the segmentation mask data can be generated. In examples, each of these features may be applied separately or together, or in any combination. That is, although in the example of Figure 5 these features are presented together for the sake of brevity, it will be appreciated that this need not necessarily be the case, and that in other examples, the method may include none or one or more, in any combination, of these features. It will also be appreciated that any one or more of the features of the example of Figure 5 may be provided in combination with any one or more of the features described above with reference to Figures 1 to 4.

Referring to Figure 5, in this example, the method comprises providing first medical imaging data 502 to a descriptor model 504 (e.g. as per any one of the examples described above). The method comprises generating, by the descriptor model 504, a first descriptor for a first location 538 in the medical imaging data (e.g. as per any one of the examples descried above). The method comprises inputting the first descriptor 506 to a trained machine learning model 560 (e.g. as per any one of the examples described above).

In the example of Figure 5, the trained machine learning network 560 comprises a residual neural network 508, a projection component 540, a plurality of classifiers 552-558 (e.g. as per any of the examples described above), and a first classifier 528. The trained machine learning model 560 may be implemented as a neural network, different layers and/or heads of which respectively corresponding to the residual neural network 508, the projection component 540, the plurality of classifiers 552-558, and the first classifier 528.

In this example, the method comprises determining, based on an input of the first descriptor 506 into the residual neural network 508, a descriptor 526 derived from the first descriptor (also referred to herein as the derived descriptor 526). Using the residual neural network 508 to determine the derived descriptor 526 (on which further steps of the method are based in this example) may help improve the optimisation of the machine learning model 508 during training. A more accurate segmentation may therefore be provided.

Specifically, in this example, the residual neural network 508 comprises one or more residual blocks 521 (only one is shown explicitly in Figure 5). Each residual block 521 comprises a projection layer 510, a first normalisation layer 512, a first linear layer 514, a second normalisation layer 516, a second linear layer 518, and a first concatenator 522. The projection layer 510 projects the first descriptor 506 to the first concatenator 522 (a so-called 'skip connection'). The projection layer 510 also projects the first descriptor 506 to the first normalisation layer 512, which performs normalisation on the projection of the first descriptor 506. Normalisation can help ensure the inputs to the subsequent layer (e.g. the first linear layer 514) have a consistent distribution, which can help reduce internal covariate shift that may occur during training. The output of the first normalisation layer 512 is provided to the first liner layer 514, which performs a linear projection on that output (e.g. where each output of the projection is a weighted sum of the inputs). The output of the first linear layer 514 is provided to the second normalisation layer 516, which performs normalisation on that output. The output of the second normalisation layer 516 is provided to the second linear layer 518, which performs a linear projection on that output. The second linear layer 518 provides its output to the first concatenator 522. The first concatenator 522 concatenates the output of the second linear layer 518 with the output of the projection layer 510. This concatenation represents the final processing step of the residual block 521. The skip connection facilitates signal propagation in both forward and backward propagation paths through the neural network, which can help improve training of the neural network 560. The residual neural network 508 may comprise multiple residual blocks (in Figure 5 a further residual block is indicated by the inclusion of a second concatenator 524).

In any case, the output of the residual neural network is a descriptor 526 derived from the first descriptor 506. In examples, the derived descriptor 526 may have a smaller dimensionality as compared to the first descriptor 506. For example, in examples, the first descriptor may be a vector having 6561 entries, whereas the derived descriptor 526 may be a vector having 144 entries. This may reduce computation in subsequent steps, and hence may further increase processing speed. Other examples are possible.

In the example of Figure 5, the derived descriptor 526 is provided to the first classifier 528. In the example of Figure 5, the method comprises determining, based on an input of the derived descriptor 528 to the first classifier 528, a probability value for each one of a plurality of class labels. For example, the first classifier 528 may be configured to output the class label probabilities for each of a plurality of class labels (e.g. organ labels) given the derived descriptor 528. For example, the first classifier 528 may comprise a plurality of layers of a neural network, specifically an input layer into which the derived descriptor 526 is input, one or more hidden layers, and an output layer from which the class label probabilities for each of a plurality of class labels (e.g. organ labels) is provided. The probabilities may represent the class label composition within the plurality of regions 530-536. For example, the probability for a given class label may represent the proportion of the space or volume within the plurality of regions 530-536 that includes that class label. For example, if the space or volume within the plurality of regions 530-536 represents half kidney and half liver, but no lung, the class label probabilities may be {liver:0.5, lung:0.0, kidney:0.5}. In examples, the first classifier 528 may have been trained based on minimisation of a regression loss between the class label probabilities predicted by the first classifier 528 for a given descriptor for a given location and ground truth class label probabilities for the plurality of regions for the given location. For example, for a given descriptor, the ground truth class label probabilities may correspond to, for each class label, the proportion of the space or volume taken up by that class label within the corresponding plurality of regions.

In any case, the first classifier 528 may output the class label probabilities for each of a plurality of class labels (e.g. organ labels) given the derived descriptor 528. If the probability for a particular class label is particularly high (e.g. {liver:0.99, lung:0.05, kidney:0.05}) then it may be inferred with a relatively high confidence that each of the plurality of regions 530-536 located relative to the first location 538 for which the first descriptor 506 was determined, has that particular class label (e.g. 'liver'). In this case, it may be efficient to assign that particular class label to the each of the plurality of regions 530-536, for example without use of the plurality of classifiers 552-558. This may be efficient because in this case the resolution of the segmentation mask will not be affected by assigning that class label to each of the plurality of the regions 530-536. However, if the probability for any particular class label is not particularly high (e.g. {liver:0.5, lung:0.3, kidney: 0.2}), then it may be inferred with a relatively high confidence that the plurality of regions 530-536 located relative to the first location 538 for which the first descriptor 506 was determined, may have a mix of different class labels. In this case, in order to maintain the resolution of the segmentation mask, the plurality of classifiers 552-558 are used to determine the class label separately for each of the plurality of regions 530-536.

Accordingly, in examples, the method may comprise determining, based on an input of the derived descriptor 528 to the first classifier 528, a probability value for each one of a plurality of class labels (e.g. {liver:0.99, lung:0.05, kidney:0.05}). The method may comprise, in response to a particular one of the determined probability values (e.g. 'liver:0.99') being greater than a threshold (e.g. 0.95), assigning the class label (e.g. 'liver') for which the particular probability value was determined to each of the plurality of regions 530-536, thereby to determine the class label for each of the plurality of regions 530-536. This scenario is represented in Figure 5 by the first classifier 528 assigning each of the plurality of regions 530-538 the class label 'A'. In this case, the method may comprise generating the segmentation mask data 570 based at least in part on the class label determined by the first classifier 528. For example, in this scenario, the value of each of the elements of the segmentation mask data 570 located in the plurality of regions 530-536 may be assigned the class label (e.g. 'liver').

However, in the example of Figure 5, responsive to each one of the determined probability values (e.g. {liver:0.5, lung:0.3, kidney: 0.2}) being less than the threshold (e.g. 0.95), the method comprises determining the class label for each one of the plurality of regions 530-536 using a respective different one of the plurality of classifiers 552-558 of the trained machine learning model 560 (e.g. as described above with reference to Figures 1 to 4). That is, where the probability for the class label having the highest probability is less than the threshold (e.g. 0.95), then the plurality of classifiers 552-558 are used to determine the class label for each region 530-536. This scenario is represented in Figure 5 by the arrow from the first classifier 528 back to the derived descriptor 526.

The use of the first classifier 528 allows for the steps associated with using the plurality of classifiers 552-558 to be skipped in cases where there is a high probability that the block of regions 530-536 has a uniform class label. This may further increase the speed of the segmentation. In examples, the plurality of class labels for which the first classifier 528 is configured to determine probabilities (e.g. 'liver', 'kidney', `lung' etc.) may be the same as the plurality of class labels that each of the plurality of classifiers 552-558 can determine for the plurality of regions 530-536 (e.g. 'liver', 'kidney', 'lung' etc.).

In the scenario where the plurality of classifiers 552-558 are to be used, the method continues as follows. The derived descriptor 526 is provided to the projection component 540. In the example of Figure 5, the method then comprises, by the projection component 540, projecting the derived descriptor 526 to each of a plurality of second descriptor spaces, thereby to determine a respective plurality of second descriptors 542, 544, 546, 548. For example, the projection component 540 may be provided by fully connected layers of a neural network. The class label for each one of the plurality of regions 530-536 is determined based on an input of a respective one of the plurality of second descriptors 542-548 into a respective one of the plurality of classifiers 552-558. For example, a particular descriptor space may correspond to a particular one of the classifiers 552-558. The particular second descriptor 542-548 that is provided by the projection of the derived descriptor 526 to that second descriptor space may be input to that particular one of the classifiers 552-558. In other words, the projection of the derived descriptor 526 to a particular one of the descriptor spaces results in a particular one of second descriptors 542-548, which is then input to a particular corresponding one of the classifiers 552-558. In the example of Figure 5, the second descriptors 542, 554, 546, 548 correspond to the classifiers 552, 54, 556, 558, respectively. The projection component 540 learns, through the training of the machine learning model, the optimal projection of the derived descriptor 526 to each second descriptor space that will ultimately optimise the determination of the appropriate class label by the corresponding classifier 552-558. This may help improve the accuracy of the segmentation.

In examples, the projection by the projection component 540 may reduce the dimensionality of the derived descriptor. For example, the projection may be a low-rank projection. For example, for each of the of the plurality of second descriptor spaces, the projection of the derived descriptor 526 by the projection component 540 to the second descriptor space reduces the dimensionality of the derived descriptor 526. As such, each of the second descriptors 542-548 may have a lower dimensionality than the derived descriptor 526 (and/or the first descriptor 506). This may reduce the amount of computation for each of the plurality of classifiers 552-558, which may in turn further increase the speed of the segmentation. As an example, the dimensionality may be reduced from 144 to 8.

In the example of Figure 5, the method comprises inputting each second descriptor 542, 544, 546, 548 to its corresponding classifier 552, 554, 556, 558, respectively. The method comprises, by each classifier 552, 554, 556, 558, classifying the input second descriptor 542, 544, 546, 548 to determine a class label for the corresponding region 530, 532, 534, 536, respectively. As such, the class label for each one of the plurality of regions 530, 532, 534, 536 is determined based on the input of a respective one of the plurality of second descriptors 542, 544, 546, 54 into a respective one of the plurality of classifiers 552, 554, 556, 558 of the trained machine learning model 560. Each classifier 552-558 may be provided by a separate classifier head in the neural network. Each classifier 552-558 may comprise a respective plurality of layers of a neural network, specifically an input layer into which the respective second descriptor 542-548 is input, one or more hidden layers, and an output layer from which the class label for the respective region 530-536 is provided. In examples, for a given second descriptor input, each classifier 552-558 may determine, for each of a plurality of possible class labels (e.g. organ labels, such as 'liver', 'kidney', `lung', 'heart' etc.) a probability that the corresponding region 530-536 of the first medical imaging data represents that class (e.g. organ). The class label with the highest probability may be determined as the class label for that region 530-536. In the example of Figure 5, the classifiers 552, 554, 556, 558 classify the second descriptors 542, 544, 546, 548 to determine the class labels B, A, A, B, for the regions 538, 532, 534, 536, respectively. Accordingly, a class label is determined for each of the plurality of regions 530-536 independently, and a higher resolution segmentation may be provided (i.e. having the resolution of the individual regions 530-536 as compared to the resolution of the block of regions 530-536). However, since those class labels are determined based on one first descriptor 506, these class labels can be determined relatively quickly. Alternatively, or additionally, since those class labels are determined based on a plurality of independent classifiers that can work in parallel, the class labels can be determined relatively quickly.

In the example of Figure 5, the method may then comprise generating the segmentation mask data 570 based at least in part on the class label determined by the plurality of classifiers 552-558. For example, in this scenario, for each region 530-536, the value of each of the elements of the segmentation mask data 570 located in the region 530-536 may be assigned the class label determined for that region. In the example of Figure 5, in this scenario where the plurality of classifiers 552-558 are used, the elements of the segmentation mask data 570 located in the region 530, 532, 534, 536, are assigned the class label B, A, A, B, respectively.

As mentioned above, although Figure 5 illustrates an example trained machine learning model 560 comprising a number of features in combination (e.g. the plurality of classifiers 552-558 in combination with each of the residual neural network 508, the first classifier 528, and the projection component 540), it will be appreciated that this need not necessarily be the case, and that in other examples, the trained machine learning model may comprise none or one or more of the residual neural network 508, the first classifier 528, and the projection component 540. In examples where the residual neural network 508 is not included, the first descriptor 506 may be used instead of the derived descriptor 526, for example for input to the first classifier 528 and/or the projection component 540 (or in examples where there is no projection component 540, for input into each of the plurality of classifiers 552-558). In examples where the first classifier 528 is not included, the derived descriptor 526 (or in examples where there is no residual neural network 508, the first descriptor 506) may be provided directly to the projection component 540 (or in examples where there is no projection component 540, directly to the plurality of classifiers 552-558) without being provided to the first classifier 528. In examples where the projection component 540 is not included, the derived descriptor 526 (or in examples where there is no residual neural network 508, the first descriptor) may be provided to each of the plurality of classifiers 552-558. Further, although in examples it is described that the derived descriptor 526 is derived by passing the first descriptor 506 through the residual neural network, it will be appreciated that this need not necessarily be the case, and that in other examples, different or additional functions or operations may be applied to the first descriptor 506 in order to determine the derived descriptor 526. Indeed, it will be appreciated that the example of Figure 5 is one example implementation and that other example implementations may include fewer or more steps or components.

In the examples described above, a first descriptor 332, 406, 506 for a first location 204, 304, 404, 538 of the first medical imaging data 202, 302, 502 is input to the trained machine learning model 460, 560, and the class label A, B for each of a plurality of regions 430-436, 530-532 having predetermined locations relative to the first location 204, 304 538 are determined. In examples, this method may be performed for each of a plurality of different first locations in the medical imaging data 202, 302, 502, for example in parallel. For example, this may provide for the determination of segmentation mask data covering multiple blocks of the first medical imaging data 202, 302, 502, for example covering the entire first medical imaging data 202, 302, 502. Performing the method for each of a plurality of different first locations in parallel may further increase the speed with which segmentation mask data 570 is generated. As mentioned above, in examples, for a given first location 204, 304 538, the plurality of regions 430-436, 530-532 may form a block with the first location 204, 304, 538 at the centre of the block. The plurality of first locations for which the method may be performed may accordingly be chosen so that the corresponding blocks are adjacent to one another. This may help ensure that there are no gaps in the segmentation mask data. This may also help ensure that for each block of the first medical imaging data, a class label is only determined once, thereby helping to ensure a minimization of computational load and hence speed in determining the segmentation mask. For example, in the case that a block 418 of regions 430-432 is 10mm³, the method may be performed in parallel for each of a plurality of first locations separated by 10mm in all 3-Dimensions.

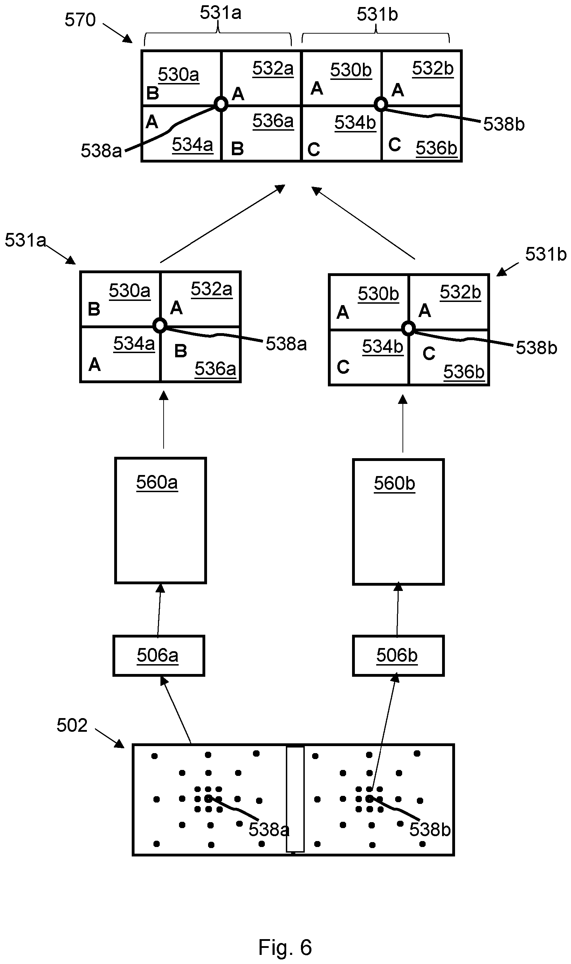

Referring to Figure 6, there is illustrated an example of performing the method according to any one of the examples described above for a plurality of first locations 538a, 538b. In this example, the method may comprise performing the steps of obtaining a first descriptor 506a and determining the class label A, B, C for each of the plurality of regions 530a-536a, 530b-536a, for each of a plurality of different first locations 538a, 538b in the first medical imaging data 502 in parallel, thereby to determine the class label for each region of a respective plurality of sets (e.g. blocks) 531a, 531b of regions 530a-536a, 530b-536b, of the first medical image data 502. In this case, generating the segmentation mask data may be based on the class labels determined for each region 530a-536a, 530b-536b of the plurality of sets (e.g. blocks) 531a, 531b of regions. Specifically, in the example of Figure 6, the method comprises obtaining a first descriptor 506a for a first location 538a in the first medical imaging data 502, and at the same time (i.e. in parallel), obtaining a first descriptor 506b for a second location 538b in the first medical imaging data 502. As described above, in examples, the first location 538a and the second location 538b may be separated by the length of one block 531a, 531b of the plurality of regions. The method then comprises, determining, based on an input of the first descriptor 506a for the first location 538a to a first instance of the trained machine learning model 560a, the class label B, A, A, B for each of a first plurality (block 531a) of regions 530a-536a of the first medical imaging data, each region 530a-536a of the first plurality 531a having a respective different predetermined location relative to the first location 538a; and at the same time (i.e. in parallel) determining, based on an input of the first descriptor 506b for the second location 538b to a second instance of the trained machine learning model 560b, the class label A, A, C, C for each of a second plurality (block 521b) of regions 530b-536b of the first medical imaging data, each region 530b-536b of the second plurality 521b having a respective different predetermined location relative to the second location 538b. The method may then comprise generating the segmentation mask data 570 based on the class labels determined for each region 530a-536a, 530b-536b of the plurality of sets (e.g. blocks) 531a, 531b of regions. For example, as shown in Figure 6, elements of the segmentation mask data that are located in the region 530a, 532a, 534a, 536a, 530b, 532b, 534b, 536b are assigned the class label B, A, A, B, A, A, C, C, respectively. The parallel processing described above may be done for any number of first locations, for example for each of a regular grid of first locations covering the entire first medical imaging data, thereby to generate segmentation mask data for the entire first medical imaging data. For example, multiple processing threads may be used to fill the entire area or volume in parallel.

Referring to Figure 7, there is illustrated a segmentation mask 702 rendered from segmentation mask data according to an example. In this example, each different segmentation class is represented by a different shade. Different regions of the segmentation mask have different class labels, and hence are shaded differently. For example, in Figure 7, regions 704 having a first class label are shaded dark grey, regions 706 having a second class label are shaded white, and regions 708 having a third class label are shaded light grey etc. Accordingly, the generated segmentation mask data can be rendered to display the segmentation mask for the first medical image for which the segmentation mask data is generated.

In the example of Figure 7, the class labels, and hence the resulting segmenting mask, represent organ classes. Specifically, for the results shown in Figure 7, the classifiers were trained to output an appropriate one of 199 different organ classes. Using example methods disclosed in herein, the experimental result shown in Figure 7 had a Dice score of 0.8. The Dice score in this context is a measure of the similarity between the segmentation mask output by an example of the method disclosed herein as shown in Figure 7, and a ground truth segmentation mask showing the ground truth class labels for each location (e.g. assigned by a medical professional). This demonstrates that the methods disclosed herein produce accurate segmentation mask data. Further, using the methods disclosed herein, the segmentation mask data of Figure 7 was generated in around 2 seconds. This is significantly faster than existing methods providing segmentation masks of a similar resolution (e.g. around 2 mm), which typically generate segmentation masks on the order of 10 seconds. This demonstrates that the methods disclosed herein provide fast generation of segmentation mask data of a given resolution (in this particular example, with a resolution of 2mm). Moreover, the generation of the segmentation mask data of Figure 7 in around 2 seconds was achieved without the use of a Graphical Processing Unit (GPU). Accordingly, methods disclosed herein may provide for fast and accurate segmentation with a relatively high resolution, without the need for the use of GPU hardware. Alternatively, or additionally, GPU hardware may be used with the methods disclosed herein to decrease the time taken to generate segmentation mask data (such as that in Figure 7) below 2 seconds. In any case, method disclosed herein provide for fast and accurate generation of relatively high resolution segmentation mask data. Increasing the speed with which the segmentation mask data is generated may, in turn, increase the utility of the segmentation mask, for example to medical professionals. For example, this may open up real-time or near real time segmentation applications. This may, in turn, increase the flexibility with which medical professionals can use the segmentation mask data, for example.

As above, in examples, the class labels, and hence the resulting segmenting mask, may represent organ classes. However, it will be appreciated that this need not necessarily be the case, and in other examples, the trained machine learning model may have been trained to output class labels that represent other classes, such as other anatomical or medical classes. Other examples are possible.

Referring to Figure 8, there is illustrated a training method for training a machine learning model. For example, the method may be used for training the trained machine learning model 460, 560, 560a, 560b according to any one of the examples described above with reference to Figures 1 to 7. In examples, the trained machine learning model 460, 560, 560a, 560b according to any one of the examples described above with reference to Figures 1 to 7, may have been trained by the training method of Figure 8.

The training method comprises, in step 802, providing a machine learning model, in step 804, providing training data, and in step 806, training the machine learning model based on the training data, thereby to provide the trained machine learning model. In examples, the machine learning model may be a neural network.

As per the trained machine learning model of the examples described above, the machine learning model provided in step 802 may be configured to perform steps comprising: based on an input of a given descriptor for a given location in given medical imaging data, determining a class label for each of a plurality of regions of the given medical imaging data, each region having a respective different predetermined location relative to the given location, the class label for each one of the plurality of regions being determined using a respective different one of a plurality of classifiers of the machine learning model. As above, each classifier may be configured to determine a class label for the corresponding region from a plurality of possible class labels, such as a plurality of possible organ labels, such as 'liver' 'kidney' 'heart', 'lung' and the like.

In examples, the training is based on supervised learning. For example, the training data provided in step 804 may comprise a plurality (for example hundreds or thousands) of training descriptors, each training descriptor being for a respective given location in given training medical imaging data, each training descriptor being representative of values of elements of the given training medical imaging data located relative to the given location according to the first predefined pattern. The training descriptors may be obtained from respective sets of training medical imaging data. The training descriptors may be provided using the same descriptor model that is used to obtain the first descriptor, as described above. The training data provided in step 804 may also comprise, for each training descriptor, a corresponding ground truth class label (e.g. organ label) for each of the plurality of regions of the given training medical imaging data. For example, the ground truth class label may be provided by annotation of the given training medical imaging data by a medical professional, although other annotation mechanisms are possible.

The training, in step 806, of the provided machine learning model based on the training data may comprise modifying parameters of the classifiers (for example weights of neurons of one or more neural network layers of each classifier) to minimize a loss function between the class labels determined by the classifiers based on the training descriptors and the corresponding ground truth class labels. For example, the loss function may comprise the cross-entropy loss between the class labels predicted by the classifiers based on input of the training descriptors and the corresponding ground truth class labels of the training data. The parameters of the classifiers (e.g. the weights of the neurons thereof) may be modified during training so as to minimise that cross entropy loss. This trains each classifier to accurately determine the class label for the corresponding region. Other loss functions may be used.

As described above, in some examples, the trained machine learning model 560 may comprise a projection component 540. In examples where the trained machine learning model 560 comprises the projection component 540, the machine learning model provided in step 802 may accordingly be configured to perform steps comprising: by a projection component of the machine learning model, projecting the given descriptor (or a descriptor derived from the given descriptor, as per the derived descriptor 526) to each of a plurality of second descriptor spaces, thereby to determine a respective plurality of second descriptors; the class label for each one of the plurality of regions being determined based on an input of a respective one of the plurality of second descriptors into a respective one of the plurality of classifiers of the machine learning model. In such examples, the training in step 806 may comprise modifying parameters of the projection component to minimise the loss function between the class labels determined by the classifiers based on the training descriptors and the corresponding ground truth class labels. In this way, both the projection component and the classifiers may be optimised so that each classifier accurately determines the class label for the corresponding region.

As described above, in some examples, the trained machine learning model 560 may comprise a first classifier 528. In examples where the trained machine learning model 560 comprises the first classifier 528, the machine learning model provided in step 802 may accordingly be configured to perform steps comprising: determining, based on an input of the given descriptor (or a descriptor derived from the given descriptor, as per the derived descriptor 526) to a first classifier, a probability value for each one of a plurality of class labels. In such examples, the training data provided in step 804 may comprise: for each training descriptor, a corresponding ground truth probability value for each of the plurality of class labels. For example, for a given training descriptor, the ground truth probability value for a given class label may be the proportion of the space or volume within the plurality of regions, corresponding to the given location, that includes that class label. In other words, the ground truth class label probability values may correspond to, for each class label, the proportion of the space or volume within the plurality of regions taken up by that class label. For example, if the space or volume within those plurality of regions represents half kidney and half liver, but no lung, the ground truth probabilities would be given by {liver:0.5, lung:0.0, kidney:0.5}. These proportions may be determined based on the class labels within the plurality of regions of given medical imaging data that has been annotated with the ground truth class labels, for example by a medical professional.

The training, in step 806, of the machine learning model may comprise modifying parameters of the first classifier to minimize a loss function between the probability values determined by the first classifier based on the training descriptors and the corresponding ground truth probability values. For example, the loss function may comprise a regression loss between the probability values determined by the first classifier based on the training descriptors and the corresponding ground truth probability values. For example, the parameters of the first classifier (e.g. the weights of the neurons thereof) may be modified during training so as to minimise the regression loss between the class label probabilities predicted by the first classifier for a given descriptor for a given location and ground truth class label probabilities for the plurality of regions for the given location. This trains the first classifier to accurately determine the probabilities for each of the plurality of class labels for a first location (and hence the composition of the class labels within the plurality of regions having predetermined locations relative to the first location). Accordingly, it can be accurately determined whether to determine the class label for each of the plurality of regions based on the output of the first classifier 528 alone, or instead based on the output of the plurality of classifiers 552-558, as described above.

As described above, in some examples, the trained machine learning model 560 may comprise a residual neural network 508. In examples where the trained machine learning model 560 comprises the residual neural network 507, the machine learning model provided in step 802 may accordingly be configured to perform steps comprising determining, based on an input of the given descriptor into a residual neural network of the machine learning model, the descriptor derived from the given descriptor. In these examples, the training in step 806 may comprise modifying parameters of the residual neural network so as to minimize the loss function between the class labels determined by the classifiers based on the training descriptors and the corresponding ground truth class labels. Alternatively, or additionally, in examples where the machine learning model comprises the first classifier, as above, the training in step 806 may comprise modifying parameters of the residual neural network so as to minimize a loss function between the probabilities determined by the first classifier based on the training descriptors and the corresponding ground truth probabilities. Accordingly, the residual neural network can be trained to determine an optimal derived descriptor to allow the plurality of classifiers to determine the appropriate class labels, and/or for the first classifier to determine the plurality of probabilities.

It will be appreciated that in some examples, the training of the machine learning model may utilise other techniques, and that in some examples, other forms of training data may be used. It will also be appreciated that the training data may, at least initially, be provided in different forms. For example, in some examples, the training data may initially be provided in the form of sets of training medical imaging data and corresponding sets of ground truth segmentation mask data. Each set of ground truth segmentation mask data may include, for each location in the corresponding training medical imaging data, the ground truth segmentation class label. For example, this may be provided by annotation by a medical professional, or in other ways. In these examples, the descriptor model 504 may be applied to the set of training medical imaging data to determine a training descriptor for a given location. Similarly, for the ground truth segmentation mask data corresponding to the set of training medical imaging data, processing may be applied to the ground truth segmentation mask data to extract the ground truth class labels (and/or the class label probabilities) for the plurality of regions corresponding to the given location. This may be repeated for each of one or more given locations, in each of a plurality of sets of training medical imaging data. In such a way, the training data comprising the following may be provided: a plurality of training descriptors, each training descriptor being for a respective given location in given training medical imaging data, each training descriptor being representative of values of elements of the given training medical imaging data located relative to the given location according to the first pattern; and for each training descriptor, a corresponding ground truth class label for each of the plurality of regions of the given training medical imaging data (and/or corresponding ground truth class label probabilities). It will be appreciated that, similarly, the training data in each of the other examples described above may, at least initially, be provided in different forms, such as in the form of sets of training medical imaging data and corresponding sets of ground truth segmentation mask data, for example as described above.

Referring to Figure 9, there is illustrated an apparatus 900. In this example, the apparatus is part of a system 901, comprising the apparatus 900, a storage device 910, and a display device 912, although it will be appreciated that this need not necessarily be the case. The apparatus 900 may be configured to perform the method according to any one of the examples described above with reference to Figures 1 to 8. The apparatus 900 may be implemented as a processing system and/or a computer. It will be appreciated that the methods according to any one of the examples described above with reference to Figures 1 to 8 are computer implemented methods, and that these methods may be implemented by the apparatus 900.

In the example of Figure 9, the apparatus 900 comprises an input interface 906, an output interface 908, a processor 902, and a memory 904. The processor 902 may be configured to perform the method according to any one of the examples described above with reference to Figures 1 to 8. The memory 904 may store instructions, for example in the form of a computer program, which, when executed by the processor 902 cause the processor 902 to perform the method according to any one of the examples described above with reference to Figures 1 to 9. The instructions may be stored on any computer readable medium, for example any non-transitory computer readable medium.

As an example, the input interface 906 may receive the first descriptor 332, 406, 506. The processor 902 may implement the method according to any of the examples described above with reference to Figures 1 to 8, and the processor 902 may output, via the output interface 908, segmentation mask data, for example according to any one of the examples described above with reference to Figures 1 to 8, or other data derived from the segmentation mask data. In some examples, the segmentation mask data (or data derived therefrom) may be transmitted to the storage device 910, for example implementing a database, so that the segmentation mask data (or data derived therefrom) is stored in the storage device 910. In some examples, the segmentation mask data (or data derived therefrom) may be transmitted to the display device 912 (such as a computer monitor) to allow a user, such as a radiologist, to review the segmentation mask (or data derived therefrom). In some examples, the segmentation mask data (or data derived therefrom) may be stored, alternatively or additionally, in the memory 904.

Although in some of the above examples, it is described that, for a given first location 204, 304, 404, 538 in the first medical imaging data, the plurality of regions 430-436, 530-536 are each rectangular (or cuboidal) in shape, and form a block surrounding the first location, it will be appreciated that this need not necessarily be the case, and that in other examples, the regions may have other shapes or arrangements relative to the first location. Nonetheless, each region has a respective different predetermined location (such as a respective different predetermined offset) relative to the first location. In examples, each region may correspond to an individual pixel or voxel of the first medical imaging data, or may correspond to a group of pixels or voxels of the first medical imaging data. It will also be appreciated that other first descriptors having other forms or arrangements to those examples described above may be used. Similarly, although in some of the above examples it is described that there are four second descriptors, four classifiers, and/or four regions for each first location, it will be appreciated that this need not necessarily be the case, and that in other examples, for each first location, there may be a plurality (i.e. any number larger than 1) of regions, a respective plurality of classifiers, and/or a respective plurality of second descriptors.

Indeed, the above examples are to be understood as illustrative examples of the invention. It is to be understood that any feature described in relation to any one example may be used alone, or in combination with other features described, and may also be used in combination with one or more features of any other of the examples, or any combination of any other of the examples. Furthermore, equivalents and modifications not described above may also be employed without departing from the scope of the invention, which is defined in the accompanying claims.

### List of Reference Signs

- 202, 302, 402, 502: first medical imaging data
- 204, 304, 404, 538: first location
- 206, 306: elements of the first descriptor
- 212, 312, 412: first predefined pattern
- 332, 406, 506: first descriptor
- 460, 560: trained machine learning model
- 442-448, 552-558: plurality of classifiers
- 430-436, 530-536: plurality of regions
- 418, 531a, 531b: set or block of regions
- 504: descriptor model
- 508: residual neural network
- 510: projection layer
- 512, 516: normalisation layer
- 514, 518: linear layer
- 521, 524: residual blocks
- 522: concatenator
- 526: derived descriptor
- 528: first classifier
- 540: projection component
- 542-548: second descriptors
- A, B, C: class labels
- 570, 702: segmentation mask data
- 704, 706, 708: regions of segmentation mask data
- 900: apparatus
- 901: system
- 902: processor
- 904: memory
- 906: input interface
- 908: output interface
- 910: storage device
- 912: display device

## Claims

1. A computer implemented method of generating segmentation mask data for first medical imaging data, the first medical imaging data comprising an array of elements having respective values and representing respective locations, the method comprising:
obtaining a first descriptor for a first location in the first medical imaging data, the first descriptor being representative of values of elements of the first medical imaging data located relative to the first location according to a first predefined pattern;
determining, based on an input of the first descriptor to a trained machine learning model, a class label for each of a plurality of regions of the first medical imaging data, each region having a respective different predetermined location relative to the first location, the class label for each one of the plurality of regions being determined using a respective different one of a plurality of classifiers of the trained machine learning model; and
generating the segmentation mask data for the first medical imaging data based on the class labels determined for the plurality of regions.

2. The method according to claim 1, wherein the method comprises:
by a projection component of the trained machine learning model, projecting the first descriptor or a descriptor derived from the first descriptor to each of a plurality of second descriptor spaces, thereby to determine a respective plurality of second descriptors; and
wherein the class label for each one of the plurality of regions is determined based on an input of a respective one of the plurality of second descriptors into a respective one of the plurality of classifiers of the trained machine learning model.

3. The method according to claim 2, wherein, for each of the of the plurality of second descriptor spaces, the projection of the first descriptor or the descriptor derived from the first descriptor to the second descriptor space reduces the dimensionality of the first descriptor or the descriptor derived from the first descriptor.

4. The method according to any one of claim 1 to claim 3, wherein the method comprises:
determining, based on an input of the first descriptor or the descriptor derived from the first descriptor to a first classifier of the trained machine learning model, a probability value for each one of a plurality of class labels;
wherein determining the class label for each one of the plurality of regions using a respective different one of the plurality of classifiers of the trained machine learning model is responsive to each one of the determined probability values being less than a threshold; and
wherein the method comprises:
in response to a particular one of the determined probability values being greater than the threshold, assigning the class label for which the particular probability value was determined to each of the plurality of regions, thereby to determine the class label for each of the plurality of regions.

5. The method according to any one of claim 2 to claim 4, wherein the method comprises:
determining, based on an input of the first descriptor into a residual neural network of the trained machine learning model, the descriptor derived from the first descriptor.

6. The method according to any one of claim 1 to claim 5, wherein the first predefined pattern is such that the density of elements represented by the first descriptor decreases with increasing distance from the first location.

7. The method according to any one of claim 1 to claim 6, wherein obtaining the first descriptor comprises:
obtaining first pattern data indicating distances from the first location of elements to be represented in first descriptor;
converting, based on scaling data indicative of the size of space that each element represents, the distances to element offsets; and
for each of the element offsets, determining the value of the element of the first medical imaging data at the element offset, thereby to obtain the first descriptor.

8. The method according to any one of claim 1 to claim 7, wherein the method comprises:
performing the steps of obtaining the first descriptor and determining the class label for each of the plurality of regions, for each of a plurality of different first locations in the first medical imaging data in parallel, thereby to determine the class label for each region of a respective plurality of sets of regions of the first medical image data; and
generating the segmentation mask data based on the class labels determined for each region of the plurality of sets of regions.

9. The method according to any one of claim 1 to claim 8, wherein the segmentation mask data comprises an array of elements each having a respective segmentation mask value and representing respective locations, wherein, for each element, the segmentation mask value represents the class label determined for the region in which the element is located.

10. The method according to any one of claim 1 to claim 9, wherein the method comprises:
storing the segmentation mask data in a storage device and/or displaying a segmentation mask rendered from the segmenting mask data on a display device.

11. The method according to any one of claim 1 to claim 10, wherein the trained machine learning model has been trained by a training method comprising:
providing a machine learning model configured to performs steps comprising:
based on an input of a given descriptor for a given location in given medical imaging data, determining a class label for each of a plurality of regions of the given medical imaging data, each region having a respective different predetermined location relative to the given location, the class label for each one of the plurality of regions being determined using a respective different one of a plurality of classifiers of the machine learning model;
providing training data comprising:
a plurality of training descriptors, each training descriptor being for a respective given location in given training medical imaging data, each training descriptor being representative of values of elements of the given training medical imaging data located relative to the given location according to the first predefined pattern; and
for each training descriptor, a corresponding ground truth class label for each of the plurality of regions of the given training medical imaging data; and
training the machine learning model based on the training data, wherein the training comprises modifying parameters of the classifiers to minimize a loss function between the class labels determined by the classifiers based on the training descriptors and the corresponding ground truth class labels.

12. The method according to claim 11, wherein the provided machine learning model is configured to perform steps comprising: by a projection component of the machine learning model, projecting the given descriptor or a descriptor derived from the given descriptor to each of a plurality of second descriptor spaces, thereby to determine a respective plurality of second descriptors; wherein the class label for each one of the plurality of regions is determined based on an input of a respective one of the plurality of second descriptors into a respective one of the plurality of classifiers of the machine learning model; and
wherein the training comprises modifying parameters of the projection component to minimise the loss function between the class labels determined by the classifiers based on the training descriptors and the corresponding ground truth class labels.

13. The method according to claim 11 or claim 12,
wherein the provided machine learning model is configured to perform steps comprising: determining, based on an input of the given descriptor, or the descriptor derived from the given descriptor, to a first classifier, a probability value for each one of a plurality of class labels;
wherein the training data comprises: for each training descriptor, a corresponding ground truth probability value for each of the plurality of class labels; and
wherein training the machine learning model comprises: modifying parameters of the first classifier to minimize a loss function between the probability values determined by the first classifier based on the training descriptors and the corresponding ground truth probability value.

14. Apparatus configured to perform the method of any one of claim 1 to claim 13.

15. A computer program which when executed by a computer causes the computer to perform the method according to any one of claim 1 to claim 13.
